# EUROPEAN PATENT APPLICATION

(11) **EP 1 026 252 A1**
(43) Date of publication of application: **09.08.2000**
(21) Application number: 00200281.4
(22) Date of filing: 27.01.2000
(51) Int. Cl.: C12N 15/86, C12N 15/40, C12N 15/38, C07K 14/18, A61K 39/12, A61K 39/245, G01N 33/53

(54) **Synthetic gene of bovine viral diarrhoea virus**

(30) Priority: 02.02.1999 EP 99200304
(71) Applicant: Akzo Nobel N.V., 6824 BM Arnhem (NL)
(72) Inventor: Gunther, Michael, 17498 Insel Riems (DE)
(74) Representative: Mestrom, Joannes Jozef Louis

(57) **Abstract**

The present invention refers to synthetic Bovine Viral Diarrhoea virus genes, live attenuated Bovine Herpesvirus recombinants carrying such genes, Bovine Herpesvirus recombinant virus particles carrying the proteins encoded by such genes, vaccines based on these recombinants, methods for the preparation of such recombinants and methods for the preparation of such vaccines and to diagnostic tools.

SEQUENCE LISTING

<110> AKZO Nobel N.V.

<120> Synthetic gene of bovine viral diarrhoea virus

<130>

<140>
<141>

<160> 16

<170> PatentIn Ver. 2.1

<210> 1
<211> 92
<212> DNA
<213> bovine viral diarrhoea virus

<220>
<221> misc_recomb
<222> (1)..(4)
<223> "5' sticky end: the indicated sequence is single
      stranded; no nucleotides present on the
      complementary strand"

<220>
<221> misc_recomb
<222> (5)..(57)
<223> "double stranded part of the oligonucleotide"

<220>
<221> misc_recomb
<222> (58)..(61)
<223> "3' sticky end: the indicated sequence is single
      stranded; nucleotides TCGA are not present in this
      fragment and only indicate that the complementary

strand contains nucleotides AGCT"

<400> 1
gatctggtac cacttagact gcaaacctga atactactat gccatagcca agaatgatag 60
agtcggccta ctaggagctg aaggcctctc ga 92

<210> 2
<211> 101
<212> DNA
<213> bovine viral diarrhoea virus

<220>
<221> misc_recomb
<222> (1)..(97)
<223> "double stranded part of the nucleotide"

<220>
<221> misc_recomb
<222> (98)..(101)
<223> "3' sticky end: the indicated sequence is single
stranded; nucleotides TCGA are not present in this
fragment and only indicate that the complementary
strand contains nucleotides AGCT"

<400> 2
ccttaccact gtttggaagg aatactcacc tgaaatgacg ctggaagaca caatggtcat 60
agcctcctgc agagaaggca aatttacata ccgctcctcg a 101

<210> 3
<211> 114
<212> DNA
<213> bovine viral diarrhoea virus

<220>
<221> misc_recomb
<222> (1)..(4)

<223> "5' sticky end: the indicated sequence is single
      stranded; the nucleotides TCGA are not present in
      this fragment and only indicate that the
      complementary strand contains nucleotides AGCT"

<220>
<221> misc_recomb
<222> (5)..(110)
<223> "double stranded part of the oligonucleotide"

<220>
<221> misc_recomb
<222> (111)..(114)
<223> "3' sticky end: the indicated sequence is single
      stranded; nucleotides TCGA are not present in this
      fragment and only indicate that the vcomplementary
      strand contains nucleotides AGCT"

<400> 3
tgcagagaag gcaaatttac ataccgctca agatgcacaa gggaagctag atatcttgca 60
attttgcatt caagagcctt accgaccagc gtggtatttg agaagcttac tcga       114

<210> 4
<211> 62
<212> DNA
<213> bovine viral diarrhoea virus

<220>
<221> misc_recomb
<222> (1)..(4)
<223> "5' sticky end: the indicated sequence is single
      stranded; no nucleotides present on the
      complementary strand"

<220>
<221> misc_recomb
<222> (5)..(58)

<223> "double stranded part of the oligonucleotide"

<220>
<221> misc_recomb
<222> (59)..(62)
<223> "3' sticky end: the indicated sequence is single
      stranded; nucleotides TCGA are not present in this
      fragment and only indicate that the complementary
      strand contains nucleotides AGCT"

<400> 4
agcttttcga gggccaaaag caagaggaca cggttgagat ggatgacaac ttcgaatctc 60
ga                                                                62

<210> 5
<211> 108
<212> DNA
<213> bovine viral diarrhoea virus

<220>
<221> misc_recomb
<222> (1)..(2)
<223> "5' STICKY END: THE INDICATED SEQUENCE IS SINGLE
      STRANDED; NO NUCLEOTIDES PRESENT ON THE
      COMPLEMENTARY STRAND"

<220>
<221> misc_recomb
<222> (3)..(104)
<223> "DOUBLE STRANDED PART OF THE OLIGONUCLEOTIDE"

<400> 5
cgaatttgga ctctgcccat gcgacgccaa gcccgtagta aaggggactt tcaatacaac 60
actgctaaat ggaccggctt tccagatggt atgcccctta gggctcga              108

<210> 6

<211> 96
<212> DNA
<213> bovine viral diarrhoea virus

<220>
<221> misc_difference
<222> (1)..(3)
<223> "5' sticky end: the indicated sequence is single
      stranded; nucleotides TCGA are not present in the
      fragment and only indicat that the complementary
      strand contains nucleotides  AGCT"

<220>
<221> misc_recomb
<222> (4)..(92)
<223> "double stranded part of the oligonucleotide"

<220>
<221> misc_recomb
<222> (93)..(96)
<223> "3' sticky end: the indicated sequence is single
      stranded; nucleotides TCGA are not present in this
      fragment and only indicate that the complementary
      strain contain nucleotides AGCT"

<400> 6
ttagggtgga cagggaccgt gagctgcatg ttagctaata gggataccct agatacagca 60
gtagtgcgga cgtataggag atatcggccg tctcga                           96


<210> 7
<211> 131
<212> DNA
<213> bovine viral diarrhoea virus

<220>
<221> misc_recomb
<222> (1)..(4)

<223> "5' sticky end: the indicated sequence is single
      stranded; no nucleotides present on the
      complementary strand"

<220>
<221> misc_recomb
<222> (5)..(127)
<223> "double stranded part of the oligonucleotide"

<220>
<221> misc_recomb
<222> (128)..(131)
<223> "3' sticky end: the indicated sequence is single
      stranded; nucleotides TCGA are not present in this
      fragment and only indicate that the complementary
      strand contains nucleotides AGCT"

<400> 7
ggccgttccc ttacaggcaa gactgcatca cccagaaggt tctgggagag gatctctata 60
actgcattct tggaggaaac tggacctgca taactggaga ccaactacaa tactcaggag 120
gatccactcg a                                                      131

<210> 8
<211> 122
<212> DNA
<213> bovine viral diarrhoea virus

<220>
<221> misc_recomb
<222> (1)..(4)
<223> "5' sticky end: the indicated sequence is single
      stranded; no nucleotides present in the
      complementary strand"

<220>
<221> misc_recomb
<222> (5)..(120)

<223> "double stranded part of the oligonucleotide"

<220>
<221> misc_recomb
<222> (121)..(122)
<223> "3' sticky end: the indicated sequence is single
      stranded; nucleotides AG are not present in this
      fragment and only indicate that the complementary
      strand contains nucleotides TC"

<400> 8
gatctacgga tccattgaat cctgcaaatg gtgcggcttt aaattccaaa gaagcgaggg 60
gttaccacac taccccattg gcaagtgcag gctgaagaat gagactggct acagattagt 120
ag                                                                122

<210> 9
<211> 91
<212> DNA
<213> bovine viral diarrhoea virus

<220>
<221> misc_recomb
<222> (1)..(4)
<223> "5' sticky end: the indicated sequence is single
      stranded; no nucleotides present on the
      complementary strand"

<220>
<221> misc_recomb
<222> (5)..(87)
<223> "double stranded part of the oligonucleotide"

<220>
<221> misc_recomb
<222> (88)..(91)
<223> "3' sticky end: the indicated sequence is single
      stranded; nucleotides GTAC are not present in this

fragment and only indicate that the complementary
strand contains nucleotides CATG"

<400> 9
gatctggtag acgacacctc ttgcaatata ggaggcgtgg cgatagtacc acaggggatg 60
gtaaaatgca agataggaga cacaattgta c 91

<210> 10
<211> 84
<212> DNA
<213> bovine viral diarrhoea virus

<220>
<221> misc_recomb
<222> (1)..(4)
<223> "5' sticky end: the indicated sequence is single
stranded; no nucleotide sequences present on the
complementary strand"

<220>
<221> misc_recomb
<222> (5)..(80)
<223> "double stranded part of the oligonucleotide"

<220>
<221> misc_recomb
<222> (81)..(84)
<223> "3' sticky end: the indicated sequence is single
stranded; no nucleotides present on the
complementary strand"

<400> 10
gatctctgta caggtcatag ctcttgacac caaacttggg cctatgccct gcaagccata 60
tgagatcatt tcaagcgagg ggcc 84

<210> 11

<211> 76

<212> DNA

<213> bovine viral diarrhoea virus

<220>

<221> misc_recomb

<222> (1)..(4)

<223> "5' sticky end: the indicated sequence is single
      stranded; no nucleotides present on the
      complementary strain"

<220>

<221> misc_recomb

<222> (5)..(74)

<223> "double stranded part of the oligonucleotide"

<220>

<221> misc_recomb

<222> (75)..(76)

<223> "3' sticky end; the indicated sequence is single
      stranded; no nucleotides present on the
      complementary strand"

<400> 11
gatctcgggc ccgtagaaaa gacggcatgc accttcaact acacgaggac attaaagaac 60
aagtactttg agccgc                                                 76

<210> 12

<211> 114

<212> DNA

<213> bovine viral diarrhoea virus

<220>

<221> misc_recomb

<222> (1)..(4)

<223> "5' sticky end: the indicated sequence is single

stranded; no nucleotides present on the
complementary strand"

<220>
<221> misc_feature
<222> (5)..(110)
<223> "double stranded part of the oligonucleotide"

<220>
<221> misc_recomb
<222> (111)..(114)
<223> "3' sticky end: the indicated sequence is single
stranded; no nucleotides present on the
complementary strand"

<400> 12
gatctaccgc gggacagcta cttccagcaa tatatgctaa aaggagaata tcaatactgg 60
tttgacctgg aggtaaccga ccatcaccgg gattacttgg ccgaatccat atta 114

<210> 13
<211> 115
<212> DNA
<213> bovine viral diarrhoea virus

<220>
<221> misc_recomb
<222> (1)..(4)
<223> "5' sticky end: the indicated sequence is single
stranded; no nucleotides present on the
complementary strand"

<220>
<221> misc_recomb
<222> (5)..(111)
<223> "double stranded part of the oligonucleotide"

<220>

<221> misc_recomb
<222> (112)..(115)
<223> "3' sticky end: the indicated sequence is single
      stranded; no nucleotides present on the
      complementary strand"

<400> 13
gatctccata ttagtggtcg tcgtcgctct cctgggcggc agatacgtcc tctggctact 60
ggtcacatac atggttctat cagaacaaaa ggtcttaggg tgagcggccg ctcga      115

<210> 14
<211> 1122
<212> DNA
<213> bovine viral diarrhoea virus

<220>
<221> misc_feature
<222> (1)..(1122)
<223> "cDNA sequence of the BVDV strain 68"

<220>
<221> misc_difference
<222> (107)..(108)
<223> replace by "ag"

<220>
<221> misc_difference
<222> (140)..(141)
<223> replace by "CC"

<220>
<221> misc_difference
<222> (152)..(153)
<223> replace by "GC"

<220>
<221> misc_difference

<222> (155)..(156)
<223> replace by "AA"

<220>
<221> misc_difference
<222> (173)..(174)
<223> replace by "GA"

<220>
<221> misc_difference
<222> (230)..(231)
<223> replace by "GC"

<220>
<221> misc_difference
<222> (242)..(243)
<223> replace by "AG"

<220>
<221> misc_difference
<222> (245)..(246)
<223> replace by "AG"

<220>
<221> misc_difference
<222> (251)..(252)
<223> replace by "TC"

<220>
<221> misc_difference
<222> (257)..(258)
<223> replace by "GC"

<220>
<221> misc_difference
<222> (278)..(279)
<223> replace by "TT"

<220>
<221> misc_difference
<222> (419)..(420)
<223> replace by "GC"

<220>
<221> misc_difference
<222> (473)..(474)
<223> replace by "GA"

<220>
<221> misc_difference
<222> (477)..(478)
<223> replace by "TC"

<220>
<221> misc_difference
<222> (479)..(480)
<223> replace by "GC"

<220>
<221> misc_difference
<222> (482)..(483)
<223> replace by "CG"

<220>
<221> misc_difference
<222> (512)..(513)
<223> replace by "AG"

<220>
<221> misc_difference
<222> (515)..(516)
<223> replace by "AG"

<220>
<221> misc_difference
<222> (524)..(525)

<223> replace by "GA"

<220>
<221> misc_difference
<222> (542)..(543)
<223> replace by "GC"

<220>
<221> misc_difference
<222> (566)..(567)
<223> replace by "GC"

<220>
<221> misc_difference
<222> (575)..(576)
<223> replace by "GA"

<220>
<221> misc_difference
<222> (599)..(600)
<223> replace by "GA"

<220>
<221> misc_difference
<222> (602)..(603)
<223> replace by "CC"

<220>
<221> misc_difference
<222> (614)..(615)
<223> replace by "GC"

<220>
<221> misc_difference
<222> (617)..(618)
<223> replace by "AA"

<220>

<221> misc_difference
<222> (623)..(624)
<223> replace by "GC"

<220>
<221> misc_difference
<222> (626)..(627)
<223> replace by "GC"

<220>
<221> misc_difference
<222> (635)..(636)
<223> replace by "TT"

<220>
<221> misc_difference
<222> (644)..(645)
<223> replace by "GC"

<220>
<221> misc_difference
<222> (677)..(678)
<223> replace by "GC"

<220>
<221> misc_difference
<222> (637)..(638)
<223> replace by "GC"

<220>
<221> misc_difference
<222> (767)..(768)
<223> replace by "AA"

<220>
<221> misc_difference
<222> (770)..(771)
<223> replace by "GC"

<220>
<221> misc_difference
<222> (884)..(885)
<223> replace by "GC"

<220>
<221> misc_difference
<222> (914)..(915)
<223> replace by "AC"

<220>
<221> misc_difference
<222> (917)..(918)
<223> replace by "AG"

<220>
<221> misc_difference
<222> (929)..(930)
<223> replace by "CG"

<220>
<221> misc_difference
<222> (931)..(932)
<223> replace by "CG"

<220>
<221> misc_difference
<222> (933)..(934)
<223> replace by "GG"

<220>
<221> misc_difference
<222> (938)..(939)
<223> replace by "GC"

<220>
<221> misc_difference

<222> (998)..(999)
<223> replace by "CC"

<220>
<221> misc_difference
<222> (1025)..(1026)
<223> replace by "AA"

<220>
<221> misc_difference
<222> (1039)..(1040)
<223> replace by "GC"

<220>
<221> misc_difference
<222> (1042)..(1043)
<223> replace by "GC"

<220>
<221> misc_difference
<222> (1046)..(1047)
<223> replace by "TC"

<220>
<221> misc_difference
<222> (1058)..(1059)
<223> replace by "GC"

<220>
<221> misc_difference
<222> (1061)..(1062)
<223> replace by "GC"

<220>
<221> misc_difference
<222> (1070)..(1071)
<223> replace by "TC"

<220>
<221> misc_difference
<222> (1077)..(1078)
<223> replace by "GC"

<220>
<221> misc_difference
<222> (1112)..(1113)
<223> replace by "AG"

<400> 14
   cac tta gac tgc aaa cct gaa tac tac tat gcc ata gcc aag aat gat    48
   His Leu Asp Cys Lys Pro Glu Tyr Tyr Tyr Ala Ile Ala Lys Asn Asp
   1               5                   10                  15
   aga gtc ggc cta cta gga gct gaa ggc ctt acc act gtt tgg aag gaa    96
   Arg Val Gly Leu Leu Gly Ala Glu Gly Leu Thr Thr Val Trp Lys Glu
               20                  25                  30
   tac tca cct gaa atg acg ctg gaa gac aca atg gtc ata gcc tcg tgc    144
   Tyr Ser Pro Glu Met Thr Leu Glu Asp Thr Met Val Ile Ala Ser Cys
           35                  40                  45
   aga gaa ggt aag ttt aca tac cgc tca agg tgc aca agg gaa gct aga    192
   Arg Glu Gly Lys Phe Thr Tyr Arg Ser Arg Cys Thr Arg Glu Ala Arg
       50                  55                  60
   tat ctt gca att ttg cat tca aga gcc tta ccg acc agt gtg gta ttt    240
   Tyr Leu Ala Ile Leu His Ser Arg Ala Leu Pro Thr Ser Val Val Phe
   65                  70                  75                  80
   gaa aaa ctt ttt gag ggg caa aag caa gag gac acg gtc gag atg gat    288
   Glu Lys Leu Phe Glu Gly Gln Lys Gln Glu Asp Thr Val Glu Met Asp
                   85                  90                  95
   gac aac ttc gaa ttt gga ctc tgc cca tgc gac gcc aag ccc gta gta    336
   Asp Asn Phe Glu Phe Gly Leu Cys Pro Cys Asp Ala Lys Pro Val Val
   100 105 110
   aag ggg act ttc aat aca aca ctg cta aat gga ccg gct ttc cag atg 384
   Lys Gly Thr Phe Asn Thr Thr Leu Leu Asn Gly Pro Ala Phe Gln Met
   115 120 125
   gta tgc ccc tta ggg tgg aca ggg acc gtg agc tgt atg tta gct aat 432
   Val Cys Pro Leu Gly Trp Thr Gly Thr Val Ser Cys Met Leu Ala Asn
   130 135 140
   agg gat acc cta gat aca gca gta gtg cgg acg tat agg agg tat aga 480
   Arg Asp Thr Leu Asp Thr Ala Val Val Arg Thr Tyr Arg Arg Tyr Arg
   145 150 155 160
   cca ttc cct tac agg caa gac tgc atc acc caa aaa gtt ctg ggg gag 528
   Pro Phe Pro Tyr Arg Gln Asp Cys Ile Thr Gln Lys Val Leu Gly Glu
   165 170 175
   gat ctc tat aac tgt att ctt gga gga aac tgg acc tgt ata act ggg 576
   Asp Leu Tyr Asn Cys Ile Leu Gly Gly Asn Trp Thr Cys Ile Thr Gly
   180 185 190
   gac caa cta caa tac tca gga ggc tct att gaa tcc tgt aag tgg tgt 624
   Asp Gln Leu Gln Tyr Ser Gly Gly Ser Ile Glu Ser Cys Lys Trp Cys
   195 200 205
   ggt ttt aaa ttc caa aga agt gag ggg tta cca cac tac ccc att ggc 672
   Gly Phe Lys Phe Gln Arg Ser Glu Gly Leu Pro His Tyr Pro Ile Gly
   210 215 220
   aag tgt agg ctg aag aat gag act ggc tac aga tta gta gac gac acc 720
   Lys Cys Arg Leu Lys Asn Glu Thr Gly Tyr Arg Leu Val Asp Asp Thr
   225 230 235 240
   tct tgc aat ata gga ggt gtg gcg ata gta cca cag ggg atg gta aag 768
   Ser Cys Asn Ile Gly Gly Val Ala Ile Val Pro Gln Gly Met Val Lys
   245 250 255
   tgt aag ata gga gac aca att gta cag gtc ata gct ctt gac acc aaa    816
   Cys Lys Ile Gly Asp Thr Ile Val Gln Val Ile Ala Leu Asp Thr Lys
               260                 265                 270
   ctt ggg cct atg ccc tgc aag cca tat gag atc att tca agt gag ggg    864
   Leu Gly Pro Met Pro Cys Lys Pro Tyr Glu Ile Ile Ser Ser Glu Gly
           275                 280                 285
   ccc gta gaa aag acg gca tgt acc ttc aac tac acg agg aca tta aag    912
   Pro Val Glu Lys Thr Ala Cys Thr Phe Asn Tyr Thr Arg Thr Leu Lys
       290                 295                 300
   aat aaa tac ttt gag ccc aga gac agt tac ttc cag caa tat atg cta    960
   Asn Lys Tyr Phe Glu Pro Arg Asp Ser Tyr Phe Gln Gln Tyr Met Leu
   305                 310                 315                 320
   aaa gga gag tat caa tac tgg ttt gac ctg gag gta act gac cat cac    1008
   Lys Gly Glu Tyr Gln Tyr Trp Phe Asp Leu Glu Val Thr Asp His His
                   325                 330                 335
   cgg gat tac ttg gcc gag tcc ata tta gtg gtg gtg gta gct ctc ctg    1056
   Arg Asp Tyr Leu Ala Glu Ser Ile Leu Val Val Val Val Ala Leu Leu
               340                 345                 350
   ggt ggt aga tac gtg ctc tgg tta ctg gtc aca tac atg gtt cta tca    1104
   Gly Gly Arg Tyr Val Leu Trp Leu Leu Val Thr Tyr Met Val Leu Ser
           355                 360                 365
   gaa caa aaa gtc tta ggg                                            1122
   Glu Gln Lys Val Leu Gly
       370

<210> 15
<211> 374
<212> PRT
<213> bovine viral diarrhoea virus

<400> 15
His Leu Asp Cys Lys Pro Glu Tyr Tyr Tyr Ala Ile Ala Lys Asn Asp
1               5                   10                  15

Arg Val Gly Leu Leu Gly Ala Glu Gly Leu Thr Thr Val Trp Lys Glu
            20                  25                  30

Tyr Ser Pro Glu Met Thr Leu Glu Asp Thr Met Val Ile Ala Ser Cys
        35                  40                  45

Arg Glu Gly Lys Phe Thr Tyr Arg Ser Arg Cys Thr Arg Glu Ala Arg
    50                  55                  60

Tyr Leu Ala Ile Leu His Ser Arg Ala Leu Pro Thr Ser Val Val Phe
65                  70                  75                  80

Glu Lys Leu Phe Glu Gly Gln Lys Gln Glu Asp Thr Val Glu Met Asp
                85                  90                  95

Asp Asn Phe Glu Phe Gly Leu Cys Pro Cys Asp Ala Lys Pro Val Val
            100                 105                 110

Lys Gly Thr Phe Asn Thr Thr Leu Leu Asn Gly Pro Ala Phe Gln Met
        115                 120                 125

Val Cys Pro Leu Gly Trp Thr Gly Thr Val Ser Cys Met Leu Ala Asn
    130                 135                 140

Arg Asp Thr Leu Asp Thr Ala Val Val Arg Thr Tyr Arg Arg Tyr Arg
145                 150                 155                 160

Pro Phe Pro Tyr Arg Gln Asp Cys Ile Thr Gln Lys Val Leu Gly Glu
                165                 170                 175

Asp Leu Tyr Asn Cys Ile Leu Gly Gly Asn Trp Thr Cys Ile Thr Gly
            180                 185                 190

Asp Gln Leu Gln Tyr Ser Gly Gly Ser Ile Glu Ser Cys Lys Trp Cys
        195                 200                 205

Gly Phe Lys Phe Gln Arg Ser Glu Gly Leu Pro His Tyr Pro Ile Gly
    210                 215                 220

Lys Cys Arg Leu Lys Asn Glu Thr Gly Tyr Arg Leu Val Asp Asp Thr
225                 230                 235                 240

Ser Cys Asn Ile Gly Gly Val Ala Ile Val Pro Gln Gly Met Val Lys
                245                 250                 255

Cys Lys Ile Gly Asp Thr Ile Val Gln Val Ile Ala Leu Asp Thr Lys
            260                 265                 270

Leu Gly Pro Met Pro Cys Lys Pro Tyr Glu Ile Ile Ser Ser Glu Gly
        275                 280                 285

Pro Val Glu Lys Thr Ala Cys Thr Phe Asn Tyr Thr Arg Thr Leu Lys
    290                 295                 300

Asn Lys Tyr Phe Glu Pro Arg Asp Ser Tyr Phe Gln Gln Tyr Met Leu
305                 310                 315                 320

Lys Gly Glu Tyr Gln Tyr Trp Phe Asp Leu Glu Val Thr Asp His His
                325                 330                 335

Arg Asp Tyr Leu Ala Glu Ser Ile Leu Val Val Val Val Ala Leu Leu
            340                 345                 350

Gly Gly Arg Tyr Val Leu Trp Leu Leu Val Thr Tyr Met Val Leu Ser
        355                 360                 365

Glu Gln Lys Val Leu Gly
    370

<210> 16

<211> 70

<212> DNA

<213> bovine viral diarrhoea virus

<220>

<221> CDS

<222> (10)..(69)

<220>

<221> misc_feature

<222> (1)..(70)

<223> "Pestivirus signal sequence of CSFV strain Alfort

<400> 16

ggatccacc atg gcc ctg ttg gct tgg gcg gtg ata aca atc ttg ctg tac 51

Met Ala Leu Leu Ala Trp Ala Val Ile Thr Ile Leu Leu Tyr

1               5                   10

cag cct gta gca ggg tac c 70

Gln Pro Val Ala Gly Tyr

15                  20

## Description

The present invention refers to synthetic Bovine Viral Diarrhoea virus genes, live attenuated Bovine Herpesvirus recombinants, live attenuated and inactivated Bovine Herpesvirus recombinants carrying such genes, vaccines based on these live attenuated recombinants, methods for the preparation of such live attenuated recombinants and to methods for the preparation of such vaccines.

Bovine Viral Diarrhoea virus (BVDV), a member of the *Flaviviridiae,* is a cause of congenital and enteric disease with a wide range of clinical manifestations. The disease was first described by P. Olafson et al. (Cornell Vet. 36 (1946) 205-213) as a transmissible disease of cattle with a high morbidity and low mortality. Affected cattle displayed elevated temperature, diarrhoea, and coughing. The condition was termed bovine viral diarrhoea.

Today, BVDV is considered to be a major pathogen of cattle with a world-wide economic impact (M.L. Doherty ML, Irish Veterinary Journal 40 (1986) 189-190; H.R. Frey et al., Dtsch. Tierärztl. Wschr. 94 (1987) 580-583; C.E. Ross et al., J. Am. Vet Med. Assoc. 188 (1986) 618-619). The clinical pattern of BVDV infection within a herd and consequently its impact on production depend on the interaction of several factors, including the strain of the virus, age of the cattle, immunity in the herd, and interplay of stressors.

BVD is an acute postnatal infection in seronegative immunocompetent cattle. Acute BVDV infection enhances clinical diseases caused by other pathogens and is an important component of the bovine respiratory disease complex. Postnatal exposure of immunocompetent cattle to concytopathic biotypes of BVDV causes clinical BVD of varying severity. Naturally acquired BVD is usually characterised by high morbidity, low mortality, a normal host immune response, and minimal mucosal lesions. Pyrexia, nasal discharge, and transient leukopenia are frequently seen.

The failure to respond to BVDV infection until around day 125 of gestation can result in embryonic death, resorption, and stillbirths or, more frequently, in a whole array of non fatal teratogenic effects (H.-J. Thiel et al., Fields Virology, Eds. B.N. Fields, D.M. Knipe, P.M. Howley et al., 3rd Edn., Raven Publishers Philadelphia 1996, p. 1059 ff.).

Since BVDV-infection leads to high economic losses, there is a substantial need for vaccines against BVDV.

To control the disease, inactivated vaccines are currently preferred. They induce, however, only a limited immune response which has been shown to be insufficient for protection against antigenic variants of BVDV (Bolin et al., Am. J. Vet. Res. 52 (1991) 1033-1037). Thus there is a clear need for improved, efficacious vaccines against BVDV.

However, vaccine development has been hampered because it is not known how a protective immune response can be induced by subunit or vector vaccines. First attempts to vaccinate calves using a baculovirus expressed glycoprotein E2 provided only limited protection (Bolin and Ridpath, Arch. Virol. 141 (1996) 1463-1477).

Since, as mentioned above, it is not known how a protective immune response can be induced, it is important for potential vaccines to at least mimic the natural infection as closely as possible. Therefore, in order to improve the immune response it is tempting to express the immunologically relevant BVDV E2 glycoprotein by a live recombinant vector virus.

These vector viruses mimic the natural infection in the sense that they do infect host cells and express, next to their own genetic material, the additional genetic information cloned into their genome.

An obvious vector for essaying the possible expression of the BVDV E2 glycoprotein under laboratory conditions is the vaccinia virus. This virus has successfully been used as an expression vector for a multitude of different genes for many years (see e.g. Paoletti, US 4,603,112 regarding the general principle for integration of foreign DNA into vaccinia virus to produce a modified virus capable of expressing foreign genes. For a general review about vaccinia virus vectors, see e.g. D.J. Jolly, Semin-Immunol. 2(5) (1990) 329-339).

For experimental purposes, the use of vaccinia virus as a live recombinant vector virus is attractive, because much is known about the virus and many tools for making vaccinia recombinants are available.

Vaccinia virus, however, is known to have an extremely broad host range. It is known to be infectious for all mammalian species tested so far, ranging from rabbits, mice, racoons, sheep, goats and camels to humans (D.J. Jolly, Semin-Immunol. 2(5) (1990) 329-339).

From an environmental point of view, this broad host range of live recombinant viruses in general is an unwanted situation, especially where only bovine animals are target animals for BVDV-vaccination. Vaccinia virus, although a bovine pathogen, would therefore certainly not be the vector of preference for use in animal vaccines for the protection of bovine animals against BVDV.

Promising live recombinant vector virus candidates however would be the live attenuated bovine herpesviruses (BHV), since such bovine herpesviruses, if expressing the BVDV E2-gene, would have the following advantages:
⇒ they are host-specific: they only infect bovine species;
⇒ they protect against two different diseases: Bovine herpesvirus infection and BVDV-infection.

Live attenuated recombinant BHV carrier viruses are known to be good potential expression vectors and vaccine viruses at the same time (R.S. Schrijver et al., Vaccine 15 (1997) 1908-1916; S. Chowdhury, Vet. Microbiol. 52 (1996) 13-23; F. Fehler et al., J. Virol.66 (1992) 831-839; C. Schröder et al., J. Virol. 70 (1996) 25-33; J. Schmitt et al., J. Virol. 70 (1996) 1091-1099; G.M. Keil et al., J. Virol. 70 (1996) 3032-3038; E. Hanon, J. Virol. 70 (1996) 4116-4120; J.C. Whitbeck et al., J. Virol. 70 (1996) 7878-7884; A. Miethke et al., J. Gen. Virol. 76 (1996) 1623-1635).

BHV-1 has e.g. been used for the expression of LacZ (J. Schmitt et al., J. Virol. 70 (1996) 1091-1099). BHV-4 has been used e.g. for the expression of the LacZ-gene (Vanderplasschen et al., Virology 213 (1995) 328-340), and the herpes simples virus I thymidine kinase gene (Keil, G.M., VIIIth International Congress of Virology, Berlin, 26-31 August, 1990).

Until now, however, expression of BVDV genes cloned in BHV as a live attenuated recombinant carrier virus, let alone showing in vivo protection by such a BHV-recombinant, has not been reported in the art. This is due to the fact that BHV is replicating/expressing in the nucleus, whereas BVDV is replicating/expressing in the cytoplasm.
Two main problems are known to exist when genes of a cytoplasmatically replicating virus are expressed in the nucleus, which is an evident consequence of cloning into the genome of a virus that replicates in the nucleus, e.g. BHV:
a) For genes from cytoplasmatically replicating viruses, e.g. the E2 gene of the closely related classical swine fever virus - which is also a member of the *Flaviviridia*e - it has been shown that the presence of cryptic splice signals caused unwanted splicing of E2 mRNA in the nucleus of mammalian cells. This is explained in more detail below. Consequently no expression of the E2 glycoprotein occurs (J.S. Shiu et al., J. Virol. Meth. 69 (1997) 223-230). The problem of the existence of cryptic splice sites is a general problem when expressing genes originating from cytoplasmatically replicating viruses in the nucleus.
b) Moreover, cytoplasmatically replicating viruses have a low G/C content. For genes of such viruses to be expressed successfully in the nucleus, the low G/C content of the genes to be expressed must necessarily be brought up to the high G/C level of the genes of the vector virus. This necessity was shown e.g. for the related Bovine Respiratory Syncytial Virus (BRSV; R.S Schrijver et al., Vaccine 15 (1997) 1908-1916; G. Kühnle et al., J. Virol. 72 (1998) 3804-3811). Expression of the G glycoprotein of BRSV by recombinant BHV was only possible after increasing the G+C content of the open reading frame (ORF) from 42 % to 62 % and by simultaneous removal of all potential splice donor signals. The same has been shown for the BRSV-F-gene.

Comparison of the GC-content of BVDV-genes and BHV-genes showed the following: the average GC-content of the BVDV-E2-gene is about 46 %, whereas the G/C content of e.g. the BHV-gD-gene is about 72 %. Therefore, on the basis of the prior art, it was to be expected that the GC-content of the synthetic BVDV-gene had to be raised dramatically by replacing the nucleotides A and T by G and C, as far as possible within the framework of what is allowed by the degeneracy of the genetic code. This is an arduous and labour-intensive task since first of all for each G/C-enhancing change in each codon, it must be checked if the changed codon still encodes the native amino acid. Secondly, after this has been done, the whole G/C-enriched E2 gene has to be synthetically prepared.

Surprisingly it was found now, that if the original native BVDV E2-gene is replaced by a synthetic BVDV-gene having a synthetic sequence from which only splice-sites were removed, and still encoding the naturally occurring amino acid sequence, the gene is successfully expressed in the nucleus. This is fully unexpected in view of the extreme difference between the BVDV GC-content (46 %) and the average GC-content of BHV-genes, which is above 70 %.

Therefore, one embodiment of the invention relates to synthetic BVDV-genes having a not naturally occurring nucleotide sequence, from which only splice-sites were removed but still encoding a naturally occurring amino acid sequence.

Since the E2-protein is an important immunogenic protein of BVDV, a preferred form of the invention relates to the synthetic BVDV-gene encoding the BVDV E2 protein.

The use of BHV (replicating in the nucleus) for the expression of BVDV genes, normally replicating in the cytoplasm, may lead to the formation of unstable (BVDV-)RNA due to the presence of obvious and/or cryptic splice-donor and/or -acceptor sites, that are recognised by the nuclear RNA-splicing mechanism. Splice-sites have small consensus sequences for both splice-donor sites (GTRAGT as a consensus sequence where R is A or G; splice donor sites can also be GTATGT and GTAGAT) and splice acceptor sites (YₙNYAG where Y is C or T, and N can be any nucleotide). In e.g. the nucleotide sequence of the wild-type E2-gene at least four potential splice-donor sites at positions 152-157, 419-424, 614-619, and 1061-1066 matching the AGGU consensus sequence were found, which are not present in the synthetic E2-gene.

Consensus sequences of both the splice-donor- and -acceptor as known in the art, have been mentioned above. Splice-sites can be removed by replacing one or more nucleotides of the potential splice-site within the framework of what is allowed by the degeneracy of the genetic code. This can be done by e.g. site-directed mutagenesis, or replacement of small or longer fragments by fragments with an alternative sequence. Alternatively, the whole gene can be replaced by synthetic DNA-fragments. Techniques for site-directed mutagenesis, and for DNA-synthesis are known in the art.

It may not be necessary to remove all potential splice sites, since in many cases, the removal of only a splice-donor, or splice-acceptor site will suffice to suppress splicing. Therefore, a more preferred form of this embodiment relates to the BVDV E2-gene modified in such a way that at least one possible splice-donor or acceptor site found in the naturally occurring nucleic acid sequence is removed in the synthetic BVDV-gene.

Comparison of the nucleotide sequence of the synthetic E2-gene according to the invention and the original E2-gene shows, that the GC-content of the synthetic gene is only slightly higher than the GC-content of the native E2-gene. Due to the removal of splice donor sequences the G+C content of a synthetic E2 ORF inherently increased from 46 % to approximately 48%. Nevertheless the synthetic gene of the present invention can, in spite of its very low G/C content, surprisingly efficient be expressed in BHV-recombinant viruses under the control of eukaryotic promoters, and the so obtained BHV/BVDV-recombinant viruses can be propagated in a stable manner.

Therefore, in an even more preferred form, the synthetic BVDV E2-gene according to the invention has a GC content between 46 and 60 %, or even between 48 and 50 %.

Although it is in principle possible to fully adapt the GC-content to the high GC-content of BHV, a very efficient expression was already obtained when the GC-content was 48%. Thus in a still even more preferred form, the synthetic BVDV-gene has a GC-content of approximately 48 %.

A naturally occurring nucleic acid sequence is understood to be a nucleotide sequence as it is found in naturally occurring viruses, such as the viruses isolated from the field.
The synthetic gene according to the present invention has a nucleotide sequence that, albeit different from the original nucleotide sequence (i.e. the nucleotide sequence found in virus isolated from the field), still encodes exactly the same amino acid sequence, and thus encodes the native protein. This is possible due to the degeneracy of the genetic code: all amino acids (with the exception of Met and Trp) are coded for by at least two different triplets. In the case of Leu, there are even 6 different triplets encoding this amino acid. Thus, there is a large number of possibilities to modify the nucleic acid sequence encoding a certain gene, without at the same time modifying the amino acid sequence. This principle is applicable to each and every gene and therefore also to the BVDV E2-gene.

The degeneracy of the genetic code also allowed e.g. the introduction of a number of restriction enzyme cleavage sites in the nucleic acid sequence encoding the E2-protein gene, without modifying the amino acid sequence of the E2-protein.

As an example may serve the modification of the BVDV E2-gene made from position 599 onwards, where the sequence GGCTCT was replaced by GGATCC, thus providing the BamHI restriction site at position 600 without changing the amino acids glycine and serine encoded by these two triplets.

New unique restriction enzyme cleavage sites for PstI, HindIII, EagI, BamHI, SacII, and NotI were introduced in the gene encoding the BVDV-E2 protein.

Synthetic DNA is defined as a DNA that is made by synthesis, instead of being isolated from a natural source.

This does not necessarily mean that each and every nucleotide of the DNA is synthesised. It is also possible to modify an existing DNA by replacing part thereof by a part with another nucleic acid sequence using recombinant DNA technology. The result of this modification is also considered to be a synthetic DNA.

The synthetic DNA can be made in a number of different ways, all known in the art. One useful method for modifying a nucleotide sequence, is e.g. site-directed mutagenesis. With this generally known method, modifications are made deliberately at predetermined sites. It is also possible to replace small or longer fragments by fragments with an alternative sequence. Many different techniques for DNA-manipulation are currently available. One possible method of replacing (parts of) a naturally found sequence with a synthetically made sequence, is e.g. to cut the nucleic acid sequence with restriction enzymes to remove the sequence to be replaced, followed by ligation of a synthetically made fragment with the same restriction sites.
Alternatively, the whole gene can be replaced by synthetic DNA-fragments.

The synthetic BRSV E2 gene from which a splice site has been removed, and that shows a minor raise in GC-content as depicted in the overview of replacements in the sequence of SEQ ID NO: 14 was shown to be very efficiently expressed. Therefore, in the most preferred form of this embodiment, the BVDV gene has the nucleotide sequence presented in the overview of replacements in the sequence of SEQ ID NO: 14.

It is clear, that the BVDV-gene, in order to be expressed, must be placed under the control of a promoter.

A large number of suitable promoters is known in the art, that are recognised for their very efficient level of expression. Such promoters are e.g. the Pseudorabies gX-promoter, the Pseudorabies TK-promoter, the Adenovirus Major Late promoter, the Retroviral Long Terminal Repeat, the SV40 Early and Late promoters, the Mouse Cytomegalovirus immediate early (MCMVie1) promoter, the Mouse Cytomegalovirus early (MCMVe1) promoter, the Human Cytomegalovirus immediate early (HCMVie1) promoter, and the BHV-gE promoter. All these promoters have been described and are known in the art for many years.

From these, the MCMVie1 promoter, the MCMVe1 promoter, the HCMVie1 promoter, and the BHV-gE promoter are preferred promoters.

Therefore, preferably the synthetic BVDV-gene according to the invention is placed under the control of one of the promoters of the group of promoters consisting of the MCMVie1 promoter, the MCMVe1 promoter, the HCMVie1 promoter, and the BHV-gE promoter.

Another embodiment of the invention relates to live attenuated carrier BHV viruses carrying a synthetic BVDV-gene according to the present invention.

As motivated above, BHV-viruses are the carriers of choice for BVDV-genes.

In a preferred form, as a recombinant BHV carrier, the BHV-1 virus is used. This virus is a very commonly found pathogen in cattle, also (as is the case with BVDV) causing high economical losses.
The most common manifestation of Bovine Herpes Virus-I infection is bovine rhinotracheitis which varies from a mild respiratory disease to a severe infection of the entire respiratory tract.
From an economical point of view, Infectious Bovine Rhinotracheitis (IBR) is also the most dramatic manifestation of BHV-I infection. Morbidity rate in IBR is usually close to 100%.

Therefore, using BHV-1 as the live attenuated recombinant carrier virus for carrying and expressing the BVDV-E2-gene is very efficient: vaccines based on such a live attenuated recombinant virus protect against both BVDV and BHV-1. Animals so vaccinated are protected against the two most frequently found causes of respiratory disease in cattle.

A large number of potential insertion sites can be used for the insertion of the synthetic BVDV-gene in the BHV carrier virus. The most suitable technique for such an insertion is homologous recombination, known in the art and frequently used. Preferably, the BVDV-gene is cloned in a non-essential BHV-gene, since in that case, a viable BHV-BVDV recombinant is obtained that is not disturbed in essential functions.

Several non-essential genes are known for BHV. The genes coding for the (glyco)proteins gE, TK, gI, gG, and US2 (=PrV 28k) are e.g. very suitable as integration sites.
Attenuated BHV-strains having deletions or insertions in such genes have been described i.a. in U.S.Patent No. 4,824,667, U.S.Patent No. 4,703,011, By Kit et al., in the Veterinary Record 127: 363-364 (1990), in European patent Publication EP 0.326.127 and in European Patent EP 0.587.805.

In a more preferred form, the BVDV-gene to be expressed is integrated in the gE-gene of BHV.

In an equally more preferred form, the BVDV-gene to be expressed is integrated in the gI-gene of BHV-1.

A live attenuated recombinant BHV carrier virus according to the invention may, next to one or more BVDV-genes, comprise other genes encoding antigens from micro-organisms or viruses that are pathogenic for cattle.
Therefore, in a preferred form, the present invention provides live attenuated recombinant BHV carrier viruses comprising, next to a BVDV-gene, a gene encoding an antigen from microorganisms or viruses that are pathogenic for cattle. Such genes are further referred to as "additional genes".

In a more preferred form of this embodiment, the additional gene is chosen from the group of cattle pathogens, consisting of Bovine Rotavirus, Bovine Respiratory Syncytial Virus, Parainfluenza type 3 virus, Bovine Paramyxovirus, Foot and Mouth Disease virus, and *Pasteurella haemolytica.*

Also, an additional gene may be introduced into the live attenuated recombinant BHV carrier virus according to the invention, that encodes a cytokine. Several cytokines, e.g. interferons are known to play an important role as immune modulators. Thus it may be advantageous to include genetic information for this kind of molecules into said section.

Still another embodiment of the present invention refers to vaccines for the protection of cattle against virus infection, based upon live attenuated recombinant BHV carrier virus expressing a BVDV-gene, according to the invention.
Vaccines based thereon have the advantage that they mimic the natural infection of not only BHV, but to a large extend also of BVDV, as motivated above.
Therefore, they provide protection against more than one pathogen, in this case against at least BHV-infection and BVDV-infection.

Vaccination is in many cases at least a two-step process: a first immunisation with an antigen triggers the immune response, and a second immunisation, the booster, actually enhances both the speed and the strength of the immune response. After a first vaccination, immunity against both the carrier itself and the antigens encoded by the heterologous gene carried by the carrier is triggered. This is the result of the fact that the recombinant carrier infects a cell and during viral replication both the viral proteins and the heterologous protein of the carried gene are expressed and become presented at the cell membrane. There they are detected by the immune system. A known problem however, when using viruses as live recombinant carriers is the following: antibodies against the recombinant carrier raised during first immunisation prevent a successful second round of infection, a booster, with the recombinant carrier. Thus no expression of proteins after boosting takes place, and therefore the immune system will not see the encoded proteins a second time. For the carrier virus this problem can be circumvented by just giving a higher dose of virus particles, since the viral proteins are per se present on the virus particle. In this approach the virus particles act as an inactivated vaccine and as such stimulates the immune system. Infection is however necessary for the heterologous gene to be expressed. Therefore, since no second round of infection occurs, the heterologous gene carried by the carrier is not expressed a second time. Thus no booster immunisation against the heterologous gene product will be generated.

It was now surprisingly found, that the BVDV E2 protein is incorporated into the envelope of the BHV-1 virus particles during the maturation of the virus. This is indeed surprising since BVDV-E2 lacks any herpesspecific targeting signals and therefore could not be expected to become incorporated into the envelope of the BHV-1 particle. The unexpected incorporation of BVDV-E2 in the envelope of BHV means that the problem mentioned above can be circumvented: a booster immunisation with a high dose of BHV virus particles carrying the BVDV E2 protein on their envelope causes a second immune response to be triggered against both the BHV envelope proteins and the BVDV protein on the envelope, without infection being necessary.
Therefore, the vaccine according to the invention may also comprise a recombinant BHV carrier virus particle according to the invention as such, regardless if the particle is still viable or not. This embodiment of the invention thus relates also to inactivated Recombinant BHV carrier virus particles carrying the BVDV E2 glycoprotein on their surface.
Thus the invention relates to vaccines suitable for booster vaccination that comprise inactivated BHV carrier viruses according to the invention. Moreover, the invention relates to the use of inactivated BHV carrier viruses according to the invention for the manufacture of vaccines for booster vaccination against BVDV-infection.

The vaccine according to the present invention may comprise a pharmaceutically acceptable carrier. One possible carrier is a physiological salt-solution.
Other pharmaceutically acceptable carriers are for instance the solutions in which adjuvants are provided.

The invention also relates to methods for the preparation of vaccines according to the invention. Such methods comprise i.a. the admixing of the live attenuated BHV-recombinant and/or the BHV-recombinant virus particle and a pharmaceutically acceptable carrier.

If desired, an adjuvant and possibly one or more emulsifiers such as Tween® and Span® are also incorporated in the live or inactivated vaccine according to the invention. Suitable adjuvants are for example vitamin-E acetate solubilisate, aluminum hydroxide, -phosphate or -oxide, (mineral) oil emulsions such as Bayol® and Marcol52® and saponins. Incorporation of the antigens in Iscoms is also a possible way of adjuvation.

It is advantageous to add a stabiliser to live or inactivated viruses, particularly if a dry composition of live viruses is prepared by lyophilisation. Suitable stabilisers are, for example, SPGA (Bovarnik et al., J. Bacteriology 59 (1950) 509), carbohydrates (such as sorbitol, mannitol, trehalose, starch, sucrose, dextran or glucose), proteins (such as albumin or casein), or degradation products thereof, and buffers (such as alkali metal phosphates). If desired, one or more compounds with adjuvant activity as described above can also be added.

A vaccine according to the invention may be administered by intramuscular or subcutaneous injection or via intranasal, intratracheal, oral, cutane, percutane or intracutane administration.

The natural route of infection is through the respiratory tract. Therefore, in a preferred form, the vaccine is formulated for intranasal administration. For the preparation of such a vaccine, a physiological salt solution is very attractive as a carrier, since it is isotonic with mucosal fluid

Alternatively or in addition, the DNA isolated from the live attenuated BHV-recombinant according to the present invention can be directly administered. Vaccination methods using naked DNA instead of viruses are sufficiently known in the art, and have been described i.a. by Cohen (Science 259: 1691-1692 (1993)), by Donnelly et al., The Immunologist 2: 20-26 (1993), by Pardoll (Immunity 3: 165-169 (1995)) and by Montgomery (Current Biology 5: 505-510 (1994)).

The useful effective amount to be administered in a vaccine will vary depending on the age, weight, and mode of administration. A suitable vaccine dosage can for example comprise about 10³ - 10¹⁰ pfu of the live recombinant per animal.

Another embodiment of the present invention relates to methods for the preparation of vaccines according to the invention. Such methods comprise admixing a live attenuated BHV-recombinant or an inactivated recombinant according to the present invention and a pharmaceutically acceptable carrier.

For the preparation of a live vaccine the BHV-I mutant according to the present invention can be grown on susceptible cells. Such susceptible cells are e.g. Madin Darby Bovine Kidney cells (MDBK-cells) or bovine embryonic cells.

The viruses thus grown can be harvested by collecting the tissue cell culture fluids and/or cells. The live vaccine may be prepared in the form of a suspension or may be lyophilised.

Still another embodiment of the present invention refers to methods for the preparation of live attenuated BHV-recombinants according to the invention.

Such methods comprise bringing together in a suitable host cell BHV-DNA and a vector comprising the BVDV-gene to be expressed, placed under the control of a suitable promoter and flanked by 3' and 5' flanking regions that share homology with BHV-sequences, in order to facilitate homologous recombination.
This can be done by first transfecting the suitable host cell with the vector, followed by infection with the BHV-virus. Alternatively, both the vector and the isolated viral DNA may transfected together into a suitable host cell.

A generally acknowledged problem in the field of vaccination is the following: the presence of antibodies against a certain pathogen in the serum of a host mammal indicates that the host has been infected with the pathogen, either in a virulent form (e.g. through filed infection) or an attenuated form. When an animal becomes infected with BVDV this means that antibodies against all immunogenic BVDV-proteins are induced. It is however impossible to discriminate between field-infected mammals and mammals vaccinated with a vaccine strain, regardless if this vaccine strain is live or inactivated, since antibodies against the viral proteins as such will be abundantly present.

Surprisingly the recombinant BHV-virus carrying the BVDV-E2 gene according to the present invention offers a solution to this problem as follows:
The E2 gene is expressed in vivo in all BVDV field strains and induces anti-E2 antibodies in infected animals. In addition, BVDV field strains induce significant amounts of antibodies against all other immunogenic BVDV-proteins. This implicates, that serum obtained from mammals infected with wild-type BVDV will always have antibodies against the E2-protein as well as antibodies against all other immunogenic BVDV-proteins. The BHV/BVDV-recombinant according to the present invention however can only induce antibodies against the BVDV E2 protein. Therefore mammals vaccinated with a BHV/BVDV-recombinant according to the invention, regardless the live or inactivated character, can only have antibodies against the BVDV E2 protein in their serum. Thus sera obtained from animals vaccinated with the BHV/BVDV-recombinant according to the invention will only react with the BVDV E2 protein, whereas sera obtained from animals infected with a BVDV field strain will contain antibodies against all immunogenic BVDV proteins.
Diagnostic tests based upon E2 protein and another immunogenic BVDV protein can therefore easily be used to discriminate between field-infection and vaccination with the vaccine according to the present invention.
The BHV/BVDV-recombinant according to the present invention thus is a very suitable marker vaccine, i.e. a vaccine strain that can be serologically discriminated from a field strain.
A diagnostic test for the discrimination between vaccine strains and field strains can be e.g. a simple ELISA-test, or the incubation of a Western blot comprising the BVDV proteins with serum of mammals to be tested.
Thus, in still another embodiment, the invention relates to diagnostic tests for the discrimination between sera from mammals infected with BVDV field strains and from mammals vaccinated with a vaccine according to the invention.

The invention is further illustrated by way of the following Examples.

### EXAMPLES

### Example 1.

### Construction of BVDV E2syn open reading frame (ORF) from synthetic oligonucleotides.

The cDNA sequence of the E2-gene of the BVDV strain 68 was determined (see SEQ ID NO: 14). This sequence was used as a guiding sequence to compose a synthetic gene called BVDV E2syn ORF that has many convenient restriction enzyme cleavage sites which also will facilitate the construction of a whole range of smaller and larger deletion mutants. The E2syn ORF does not contain any splice donor consensus sequences but still codes for an E2 protein with an amino acid sequence identical to the BVDV strain 68 encoded E2 protein.

To reach this goal a total of 13 fragments as given in SEQ ID NO: 1-13 were obtained by mixing equimolar amounts of complementary oligonucleotides in 10 mM tris-HCl, pH 7.5, boiled for 5 minutes and then slowly cooled down to room temperature.

Fragments with SEQ ID NO: 1-7 were cloned adjacently in pSP73 yielding plasmid pNH7. This was done by cleaving pSP73 with BglII and XhoI. Fragment 1 was ligated into this DNA via its cohesive ends resulting in Plasmid pNH1 which was cleaved with StuI and XhoI to receive fragment 2. The resulting plasmid pNH2 was cleaved with PstI and XhoI and fragment 3 was integrated to obtain pNH3 which was cleaved with HindIII and XhoI to construct pNH4 by integration of fragment 4. pNH4 was cleaved with SfuI and XhoI and received fragment 5 resulting in pNH5 that was cleaved with Bsu36I and XhoI for integration of fragment 6. The resulting plasmid pNH6 was cleaved with EagI and XhoI received fragment 7 to give plasmid pNH7.

Fragments A-F (SEQ ID NO: 8-13) were integrated in that order in pSP73 (Promega) to yield pSPCF. This was done by cleaving pSP73 with BglII and Xho. Fragment A was ligated into this DNA via its cohesive ends resulting in Plasmid pCA which was cleaved with BglII and BstXI to receive fragment B. The resulting plasmid pCB was cleaved with BglI and SacII and fragment C was integrated to obtain pCC which was cleaved with BglII and ApaI to construct pCD by integration of fragment D. pCD was cleaved with BglII and BsrGI and received fragment E resulting in pCE that was cleaved with BglII and AccI for integration of fragment F to obtain plasmid pCF.

Finally plasmid pNH7 was cleaved with BamHI and XhoI and received the purified BamHI-XhoI insert from plasmid pCF to yield plasmid pSPE2syn containing the reconstructed BVDV E2 ORF.

### Example 2

### Construction and analysis of BHV-1 BVDV-E2-recombinants

### Cloning of the MCMV promoters ie1 and e1 in pAT-gD PLUS.

In order to test the expression of the E2 gene under various conditions, it was decided to clone the gene under the control of various alternative promoters.

First of all, a multicloning site was inserted in pAT-gIV (Vector pAT-gIV has been described extensively in EP 0 663 403. It should be mentioned here that the vector pAT-gIV is also referred to as pAT-gD, due to the new nomenclature of the herpesvirus (glycoprotein-)genes) between the MCMVie2-polyA fragment and the gD-polyA fragment, and this new plasmid was named pAT-gD PLUS.

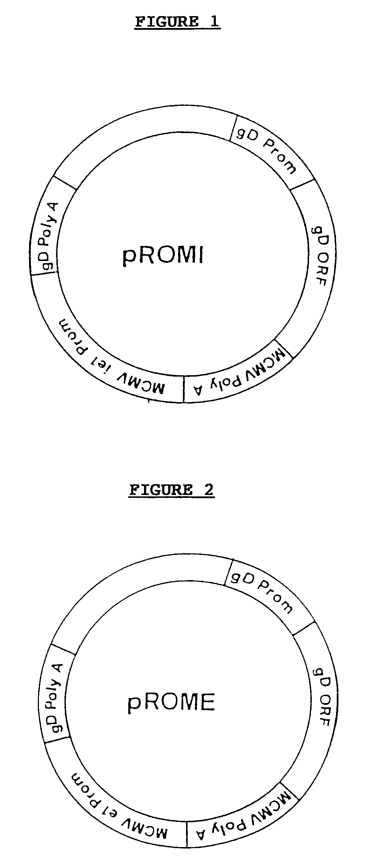

Starting with pAT-gD PLUS, two constructs were made:
Construction of pROMI: Plasmid pAMB33 (Dorsch-Häsler et al., Proc. Natl. Acad. Sci. 82 (1985) 8325-8329) was digested with HpaI and XbaI. Fragments were filled with Klenow-polymerase and isolated from agarose gels. A 1.4 kB fragment comprising the MCMV ie1 promoter was obtained.
   The plasmid pAT-gD PLUS was cleaved in its polylinker site with EcoRV. In this site, the isolated MCMV ie1 fragment was cloned, to yield pROMI (see Fig. 1).
Construction of pROME: Plasmid MM354, comprising the MCMV e1 promoter (sequence of the Murine Cytomegalovirus early 1 gene is present in the EMBL Gene Bank at Heidelberg, Germany) was digested with SmaI and HindIII. Fragments were filled with Klenow-polymerase and isolated from agarose gels. A 1.1 kB fragment comprising the MCMV e1 promoter was obtained. The plasmid pAT-gD PLUS was cleaved in its polylinker site with EcoRV. In this site, the isolated MCMV e1 fragment was cloned, to yield pROME (See Fig. 2).

### Cloning of pROMEsig.

To provide a signal peptide for correct transport and processing of BVDV E2, the pestivirus signal sequence of CSFV strain Alfort (See SEQ ID NO: 16) was ligated into pROME (see above) after cleavage with BamHI and KpnI to give plasmid pROMEsig.
Cloning of the BVDV E2syn ORF into pROMEsig. Plasmid pROMEsig was cleaved with KpnI and NotI and used for the integration of the BVDV E2syn ORF cleaved out of plasmid pSPE2syn with KpnI and NotI. The resulting plasmid was named pROME2syn.
Integration of pROME2syn into BHV-1. MDBK cells were transfected with 5 mg pROME2syn and 2.5 mg of purified DNA of gD negative virus BHV-1/80-221 (Fehler et al., J.Virol. 66 (1992) 831-839). Transfected cells were shocked with glycerol 4 hours after addition of the DNA. After development of CPE the cell culture supernatant was titrated on MDBK cells. Under these conditions only genotypically gD positive virions can grow productively and form plaques on monolayers. At 3 days p.i. virions from single plaques were isolated and DNA from MDBK cells was purified, cleaved with HindIII, size separated by agarose gel electrophoresis and transferred to nitrocellulose filters which were hybridised to ³²P-labeled probes either from the gD ORF, the lacZ ORF, the E2syn ORF or sequences downstream the E2syn ORF presented for homologous recombination. The results demonstrated that the isolated virions lack the β-galactosidase sequences and that the BVDV E2syn ORF was integrated. The resulting virus BHV-1/E2syn was plaque purified twice and used for further charactererization.

### Identification and characterization of BHV-1 expressed BVDV E2.

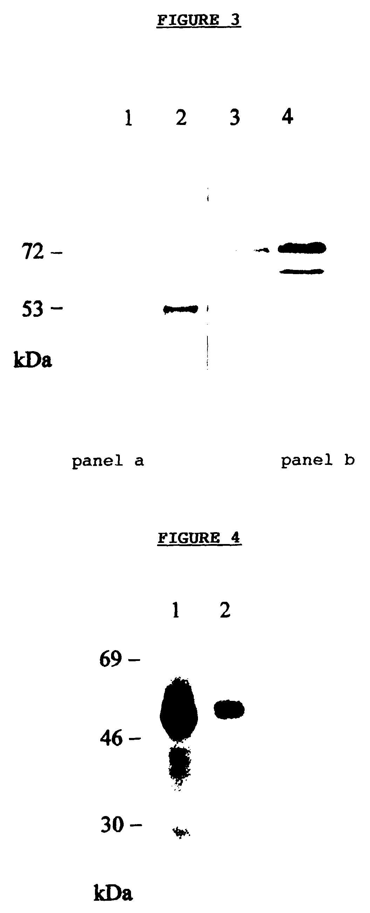

To demonstrate expression by recombinant BHV-1 and to identify the BVDV E2syn ORF encoded gene product proteins from wild type BHV-1 strain Schönböken (Fig. 3, lanes 1 and 3) and recombinant BHV-1/E2syn (Fig. 3, lanes 2 and 4), infected MDBK cells were analysed by immunoblotting using an immune serum from a BVDV strain 68 infected calf (panel a) or a rabbit serum directed against BHV-1 glycoprotein D. The anti-BVDV serum did not specifically react with proteins from BHV-1 strain Schönböken infected cells but bound to a 53 kDa apparent molecular mass protein among BHV-1/E2syn infected cell proteins. This apparent molecular mass corresponds very well to the 53 kDa apparent molecular mass reported for the E2 glycoprotein expressed in BVDV infected cells. Binding of the anti-gD serum to the 72 and 68 kDa apparent molecular mass forms of BHV-1 gD (panel b) demonstrates comparable infection with BHV-1 strain Schönböken and BHV-1/E2syn, respectively.

Fig. 4 demonstrates incorporation of the BHV-1/E2syn expressed E2 glycoprotein into recombinant virions. For this purpose MDBK cells were infected with BHV-1/E2syn in the presence of ³⁵S-methionine. Proteins from lysed cells (lane 1) and purified virions (lane 2) were immunoprecipitated using the anti BVDV serum and bound proteins were visualised by fluorography after size separation in SDS-polyacrylamide gels. The result shows that the E2 glycoprotein is associated with BHV-1/E2syn virions.

### LEGENDS TO THE FIGURES

Fig. 1 shows a map of pROMI
Fig. 2 shows a map of pROME
Fig. 3 demonstrates expression by recombinant BHV-1 and identifies the BVDV E2syn ORF encoded gene product.

MDBK cells were infected with wild type BHV-1 strain Schönböken (Fig. 3, lanes 1 and 3) and recombinant BHV-1/E2syn (Fig. 3, lanes 2 and 4), and their protein content analysed by immunoblotting using an immune serum from a BVDV strain 68 infected calf (panel a) or a rabbit serum directed against BHV-1 glycoprotein D (panel b).

Fig. 4 demonstrates incorporation of the BHV-1/E2syn expressed E2 glycoprotein into recombinant virions. MDBK cells were infected with BHV-1/E2syn in the presence of ³⁵S-methionine. Proteins from lysed cells (lane 1) and purified virions (lane 2) were immunoprecipitated using the anti BVDV serum and bound proteins were visualised by fluorography after size separation in SDS-polyacrylamide gels. The result shows that the E2 glycoprotein is associated with BHV-1/E2syn virions.

## Claims

1. Synthetic BVDV-gene, characterised in that said BVDV-gene has a not naturally occurring nucleotide sequence encoding a naturally occurring amino acid sequence.

2. Synthetic BVDV-gene according to claim 1, characterised in that said BVDV-gene encodes the BVDV E2 protein.

3. Synthetic BVDV-gene according to claim 1 or 2, characterised in that in said BVDV-gene at least one naturally occurring putative splice-donor- or -acceptor site is removed.

4. Synthetic BVDV-gene according to claim 2 or 3, characterised in that said BVDV gene has a GC-content between 48 and 60 %, preferably between 48 and 50% %.

5. Synthetic BVDV-gene according to claim 4, characterised in that said BVDV gene has a GC-content of approximately 48 %.

6. Synthetic BVDV-gene according to claim 2, characterised in that said BVDV gene has the nucleic acid sequence presented in the overview of replacements in the sequence of SEQ ID NO: 14.

7. Synthetic BVDV-gene according to claims 1 to 6, characterised in that said BVDV gene is under the control of the MCMVie1-promoter, the MCMVe1-promoter, the HCMVie1-promoter or the BHV gE-promoter.

8. Live attenuated recombinant BHV carrier virus, characterised in that it carries a synthetic BVDV-gene according to claims 1 to 7.

9. Live attenuated recombinant BHV carrier virus according to claim 8, characterised in that said BHV-recombinant is a BHV-1 recombinant.

10. Live attenuated recombinant BHV carrier virus according to claim 9, characterised in that the synthetic BVDV-gene is inserted in the gE-gene of said BHV recombinant.

11. Live attenuated recombinant BHV carrier virus according to claim 9, characterised in that the synthetic BVDV-gene is inserted in the gI-gene of said BHV recombinant.

12. Live attenuated recombinant BHV carrier virus according to claims 8 to 11, characterised in that it comprises an additional gene encoding an antigen from a micro-organism or virus that is pathogenic for cattle.

13. Live attenuated recombinant BHV carrier virus according to claim 12, characterised in that the gene is chosen from the group of cattle pathogens, consisting of Bovine Rotavirus, Bovine Viral Diarrhoea virus, Parainfluenza type 3 virus, Bovine Paramyxovirus, Foot and Mouth Disease virus, Bovine Respiratory Syncytium virus and *Pasteurella haemolytica.*

14. Inactivated Recombinant BHV carrier virus particle, characterised in that it carries the BVDV E2 glycoprotein on its surface.

15. Vaccine for the protection of cattle against virus infection, characterised in that said vaccine comprises a live attenuated recombinant BHV carrier virus according to claims 8 to 13 and/or the DNA thereof and/or a BHV-recombinant virus particle according to claim 14 and a pharmaceutically acceptable carrier.

16. Vaccine according to claim 15, characterised in that it is formulated for intranasal administration.

17. Method for the preparation of a vaccine according to claim 15 or 16, characterised in that said method comprises the admixing of a live attenuated BHV-recombinant according to claims 8 to 13 and/or the DNA thereof and/or a BHV-recombinant virus particle according to claim 14 and a pharmaceutically acceptable carrier.

18. Method for the preparation of a live attenuated BHV-recombinant according to claims 8 to 13, said method comprising bringing together in a suitable host cell isolated BHV-DNA and a vector comprising the synthetic BVDV-gene, placed under the control of a suitable promoter and flanked by 3' and 5' flanking regions that share homology with BHV-sequences.

19. Method for the preparation of a live attenuated BHV-recombinant according to claims 8 to 13, said method comprising bringing into a suitable host cell a vector comprising the synthetic BVDV-gene, placed under the control of a suitable promoter and flanked by 3' and 5' flanking regions that share homology with BHV-sequences, followed by infection of said suitable host cell with BHV.

20. Diagnostic test for the discrimination between sera from cattle infected with BVDV field strains and cattle vaccinated with a vaccine according to claim 15 or 16, said test being characterised in that the test comprises purified BVDV E2 protein.

21. Use of inactivated BHV carrier viruses according to the invention for the manufacture of vaccines for booster vaccination against BVDV-infection.
